(19) Europäisches Patentamt
European Patent Office
Office européen des brevets

(11) Veröffentlichungsnummer: **0 160 962**
**B1**

(12) # EUROPÄISCHE PATENTSCHRIFT

(45) Veröffentlichungstag der Patentschrift: **13.09.89**

(21) Anmeldenummer: **85105543.4**

(22) Anmeldetag: **07.05.85**

(51) Int. Cl.⁴: **C 07 C 69/65,** C 07 C 67/14, C 07 C 79/46, A 01 N 37/10 // C07C57/30, C07C57/72, C07C121/52, C07C21/24

(54) **Substituierte Phenylessigsäurejodpropargylester, ihre Herstellung und Verwendung als biozide Mittel.**

(30) Priorität: **07.05.84 DE 3416772**

(43) Veröffentlichungstag der Anmeldung: **13.11.85 Patentblatt 85/46**

(45) Bekanntmachung des Hinweises auf die Patenterteilung: **13.09.89 Patentblatt 89/37**

(84) Benannte Vertragsstaaten: **AT BE CH DE FR GB IT LI NL SE**

(56) Entgegenhaltungen: **WO-A-84/00162** **GB-A-2 016 457**

(73) Patentinhaber: **THE DOW CHEMICAL COMPANY, 2030 Dow Center Abbott Road P.O. Box 1967, Midland Michigan 48640- 1967 (US)**

(72) Erfinder: **Staiger, Gerhard, Dr. Dipl.- Chem., Staudingerstrasse 65, D-8000 München 83 (DE)**
Erfinder: **Selmayr, Tassilo, Dr. Dipl.- Chem., Aberlestrasse 27, D-8050 Freising (DE)**
Erfinder: **Kinzel, Peter, Dipl.- Ing., Mareisring 30, D-8152 Feldkirchen- Westerham (DE)**
Erfinder: **Reutter, Anneliese, Dipl.- Ing., Hirschenweg 10, D-8011 Englharting (DE)**

(74) Vertreter: **Huber, Bernhard, Dipl.- Chem., Patentanwälte H. Weickmann, Dr. K. Fincke F.A. Weickmann, B. Huber Dr. H. Liska, Dr. J. Prechtel Möhlstrasse 22 Postfach 860 820, D-8000 München 86 (DE)**

**EP 0 160 962 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft am Phenylkern und/oder in Benzylstellung substituierte Phenylessigsäurejodpropargylester.

Gemäß US-PS-4 259 350 ist Phenylessigsäurejodpropargylester als biozides Mittel bereits bekannt geworden. Es wurde nun gefunden, daß eine Auswahl von im Phenylkern und/oder im Benzylstellung substituierter Phenylessigsäurejodpropargylester erhöhte fungizide Wirksamkeit aufweisen. Insbesondere zeichnen sich die erfindungsgemäßen Verbindungen durch ein breites Wirkungsspektrum aus.

Gegenstand der Erfindung sind Verbindungen der allgemeinen Formel

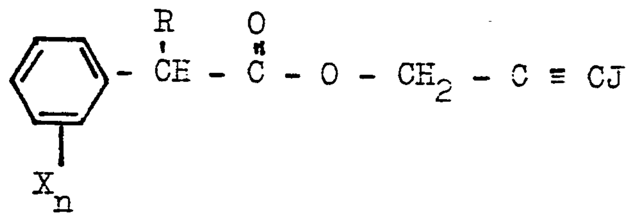

wobei

R Wasserstoff, Fluor, Chlor, Brom, Jod, Methoxy, 1-Imidazolyl und 1.2.4-Triazolyl bedeutet,
X für einen oder mehrere gleiche oder verschiedene Substituenten steht mit der Bedeutung Fluor, Chlor, Brom, Jod, Cyano, Nitro, Carboxy, Alkyl mit 1 bis 12 Kohlenstoffatomen, Alkoxy mit 1 bis 12 Kohlenstoffatomen, Cycloalkyl mit 3 bis 6 Kohlenstoffatomen, Formyl, Acetyl, Propionyl, Benzoyl, Phenylsulfonyl, Phenyl, Phenoxy und substituiertes Phenyl und Phenoxy mit jeweils 1 bis 3 Substituenten aus der Gruppe Fluor, Chlor, Brom, Nitro, Methyl und Methoxy,
n eine ganze Zahl von 1 bis 5 ist und
n mit der Maßgabe, daß R nicht Wasserstoff bedeutet, O sein kann.

Vorzugsweise ist n 1, 2, 3 oder 4.

Der bevorzugte Substitutionstyp für n = 1 ist die Parasubstitution. Weitere Beispiele sind die o- und die m-Substitution.

Für n = 2 ist die o/p-, die m/m'- und die o/o'-Substitution bevorzugt.

Für n = 3 ist die o/o'/p- und die o/m/m'-Substitution bevorzugt.

Weiterhin sind solche Verbindungen bevorzugt, bei denen X für Methyl, Nitro, Chlor, Brom, Jod und Methoxy steht.

R ist bevorzugt Wasserstoff, Chlor und Brom.

Beispiele für besonders bevorzugte Verbindungen sind:

4-Nitrophenylessigsäurejodpropargylester
2-Chlor-3.5-dinitrophenylessigsäurejodpropargylester
2.4-Dichlor-3.5-dinitrophenylessigsäurejodpropargylester
2.6-Dichlor-3.5-dinitrophenylessigsäurejodpropargylester
4-Chlor-3.5-dinitrophenylessigsäurejodpropargylester
4-Chlorphenylessigsäurejodpropargylester
α-Brom-4-chlorphenylessigsäurejodpropargylester
α-Chlor-Phenylessigsäurejodpropargylester
2.4.6-Trimethylphenylessigsäurejodpropargylester.

Weitere Beispiele für erfindungsgemäße Verbindungen sind:

4-Methoxy-phenylessigsäurejodpropargylester
4-Methyl-phenylessigsäurejodpropargylester
4-Ethyl-phenylessigsäurejodpropargylester
4-Jod-phenylessigsäurejodpropargylester
4-Brom-phenylessigsäurejodpropargylester
4-Fluor-phenylessigsäurejodpropargylester
4-Isopropyl-phenylessigsäurejodpropargylester
4-Isopropoxy-phenylessigsäurejodpropargylester
4-n-Hexyl-phenylessigsäurejodpropargylester
4-Phenyl-phenylessigsäurejodpropargylester
4-Cyclohexyl-phenylessigsäurejodpropargylester
4-Phenoxy-phenylessigsäurejodpropargylester
3-Phenoxy-phenylessigsäurejodpropargylester
2-Phenoxy-phenylessigsäurejodpropargylester
4-Carboxy-phenylessigsäurejodpropargylester

1-Chlor-phenylessigsäurejodpropargylester
2-Chlor-phenylessigsäurejodpropargylester
2.4-Dibrom-phenylessigsäurejodpropargylester
2.3.6-Trichlor-phenylessigsäurejodpropargylester
4-Cyano-phenylessigsäurejodpropargylester
α-(1.2.4-Triazolyl)-phenylessigsäurejodpropargylester
α-(1.2.4-Triazolyl)-4-Chlor-phenylessigsäurejodpropargylester
α-Imidazolyl-phenylessigsäurejodpropargylester
α-Imidazolyl-4-Chlor-phenylessigsäurejodpropargylester
4-Formyl-phenylessigsäurejodpropargylester
4-Acetyl-phenylessigsäurejodpropargylester
4-Phenylsulfonyl-phenylessigsäurejodpropargylester

Die erfindungsgemäßen Verbindungen sind durch Veresterung von 3-Jod-propinol mit entsprechend substituierten Phenylessigsäuren, bzw. in Bezug auf die Veresterungsreaktion reaktionsfreudigen substituierten Phenylessigsäurederivaten zugänglich.

Ein bevorzugtes Verfahren zur Herstellung der erfindungsgemäßen Verbindungen ist dadurch gekennzeichnet, daß Verbindungen der Formel

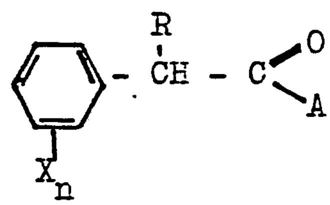

wobei
R, X und n die vorstehend gegebene Bedeutung besitzen und
A für Chlor, Brom, Jod, Acyloxy und Alkoxycarbonyloxy steht,
mit 3-Jodpropinol in Gegenwart eines Säurebindemittels umgesetzt werden.

Beispiele für A, soweit A für Acyloxy- und Alkoxycarbonyloxy-Gruppen steht, sind die 2.2-Dimethylpropionyloxy-, die Benzoyloxy-Gruppe; die Methoxycarbonyloxy- und die Ethoxycarbonyloxy-Gruppe.

Als Säurebindemittel werden insbesondere tertiäre Amine wie Triäthylamin, Dimethylcyclohexylamin, Pyridin u.a. eingesetzt. Weitere Beispiele für Säurebindemittel sind u.a. anorganische Basen wie Natriumhydroxid, Kaliumhydroxid, Calciumoxid, Magnesiumoxid, Natriumcarbonat und Calciumcarbonat.

Die Reaktionstemperaturen liegen im Bereich von -10°C bis 100°C, insbesondere 20°C bis 60°C.

Die Umsetzungen werden zumeist in inerten Lösungsmitteln wie Benzol, Toluol, Petroläther, Methylenchlorid, Chloroform, Dioxan, Diäthyläther und dgl. durchgeführt.

Üblicherweise wird so vorgegangen, daß das Phenylessigsäurederivat einer Vorlage aus Jod-propinol und Säurebindemittel bei der gewünschten Temperatur unter Rühren zudosiert wird.

Die als Ausgangsverbindungen einzusetzenden substituierten Phenylessigsäuren sind, wenn sie nicht ohnehin im Handel erhältlich sind, nach an sich bekannten Methoden zugänglich:

Beispielsweise können die gewünschten Phenylessigsäuren über die Umsetzung der entsprechend substituierten Benzylhalogenide mit Cyanid und anschließende Verseifung des Benzylcyanids erhalten werden.

Die Benzylcyanide sind vorteilhaft durch phasentransferkatalysierte Reaktion der Benzylhalogenide mit Alkalicyanid-Lösungen in einer organisch/wäßrigen 2-Phasen-System in Gegenwart von Phasentransfer-Katalysatoren wie quartären Ammonium- bzw. Phosphoniumsalzen oder Kronenäthern zugänglich.

Die Benzylcyanide werden zumeist im saueren Medium zur entsprechenden Säure hydrolysiert. Lediglich für den Fall, daß säurelabile Phenylreste vorliegen, empfiehlt sich die basische Verseifung des Cyanids.

Zur Herstellung der entsprechend substituierten Benzylhalogenide kann eine Vielzahl von Synthesewegen beschritten werden.

Beispielhaft genannt sei die an sich bekannte Chlorierung entsprechende Substituenten aufweisender Toluolverbindungen in Benzylstellung; die Chlormethylierung entsprechende Substituenten aufweisenden Benzols mit Chlorwasserstoff und Formaldehyd in Gegenwart von Zinkchlorid oder die Chlormethylierung mit alpha, alpha'-Dichlordimethyläther in Gegenwart von konzentrierter Schwefelsäure.

Ein weiterer Syntheseweg für erfindungsgemäß einzusetzende Ausgangsverbindungen eröffnet sich durch Substituierungsreaktionen an Phenylessigsäure, wobei insbesondere Nitrierungen und Acylierungen in Frage kommen. Für die Nitrierungsreaktionen eignen sich insbesondere konzentrierte Salpetersäure und Mischungen von konzentrierter Salpetersäure und konzentrierter Schwefelsäure. Oftmals wird bei diesen Reaktionen mit Vorteil in Gegenwart inerter Verdünnungsmittel, wie beispielsweise Halogenkohlenwasserstoffen oder Nitromethan, gearbeitet.

Solche erfindungsgemäß einzusetzenden Ausgangsverbindungen, bei denen der sich in Benzylstellung befindliche Rest R eine andere Bedeutung als Wasserstoff besitzt, sind in an sich bekannter Weise durch alpha-Chlorierung bzw. alpha-Bromierung der entsprechenden Phenylessigsäuren nach Hell-Volhardt-Zelinski

zugänglich. Die so erhaltenen in alpha-Stellung zur Carbonylfunktion halogenierten Phenylessigsäuren werden zur Einführung der Substituenten Fluor, Jod, Methoxy, 1-Imidazolyl oder 1.2.4-Triazolyl noch einer weiteren nucleophilen Austauschreaktion unterworfen. Oftmals kann auch Mandelsäure mit Vorteil als Ausgangsverbindung verwendet werden.

Die erfindungsgemäßen Verbindungen weisen insektizide, akarizide und insbesondere fungizide Wirksamkeit auf. Sie eignen sich insbesondere zur Bekämpfung pilzlichen Befalls an Pflanzen oder pflanzlichen Produkten.

Die erfindungsgemäßen Wirkstoffe zeichnen sich durch ein breites Wirkungsspektrum aus und eignen sich insbesondere zur Bekämpfung von Botrytis cinerea, Alternaria-Arten, Septoria-Arten, Verticillium dahliae, Penicillium glaucum, Colletotrichum-Arten, Monilia-Arten sowie Fusarium-Arten. Weiterhin sind die erfindumgsgemäßen Wirkstoffe erfolgreich einsetzbar gegen phytopathogene Pilze, die dem Saatgut anhaften, wie z. B.Tilletia tritici, Fusarium nivale und Helminthosporium-Arten.

Die erfindungsgemäßen Wirkstoffe eignen sich, ohne daß ihr Anwendungsgebiet etwa darauf beschränkt wäre, z. B. zum Einsatz im Weinbau, im Gartenbau, insbesondere im Gemüse- und Zierpflanzenbau, beim Zierrasen, beim Rapsanbau, beim Hopfenanbau, in Erdbeerpflanzungen sowie im Kernobstbau. Die erfindungsgemäßen Wirkstoffe sind zudem zur Konservierung geernteter Früchte verwendbar. Als weiteres Anwendungsgebiet wurde der Einsatz als Saatgutbeizmittel gefunden.

Die erfindungsgemäßen Wirkstoffe können allein oder im Gemisch mit anderen pestiziden, insbesondere fungiziden Mitteln, ausgebracht werden. Hervorzuheben sind Wirkstoffkombinationen mit bekannten Botrytiziden, bei denen bereits Resistenzerscheinungen festgestellt wurden.

Im allgemeinen werden die erfindungsgemäßen Wirkstoffe als Mischungen mit festen oder flüssigen Verdünnungsmitteln oder als Lösungen in festen oder flüssigen Lösungsmitteln verwendet, mit Wirkstoffgehalten von 0,01 bis 95 Gew.-%.

Die Mischungen bzw. Lösungen werden z. B. als Emulsionskonzentrate, Pasten, Spritzpulver, Granulate oder Mikrokapseln hergestellt.

Emulsionskonzentrate und Pasten enthalten im allgemeinen 10 - 60 Gew.-%, vorzugsweise 15 - 40 Gew.-%, Wirkstoff, 2 - 25 Gew.-% Dispergierhilfsstoffe und organische Lösungsmittel und/oder Wasser.

Spritzpulver enthalten meistens 10 - 80 Gew.-%, vorzugsweise 15 - 70 Gew.-%, Wirkstoff, 1 - 10 Gew.-% Dispergierhilfsstoffe und 10 - 89 Gew.-% inerte Bestandteile.

Granulate und Stäubemittel enthalten neben inerten Bestandteilen, Bindemitteln und/oder Überzugsstoffen 1 - 10 Gew.-%, insbesondere 5 - 10 Gew.-%, Wirkstoff.

Erfindungsgemäß angewandt werden:

als Dispergierhilfsstoffe, z. B. Alkyl- und Arylsulfonate, Methylcellulose, polymere Sulfonsäuren und deren Salze, Polyalkohole, Fettsäureester, Fettalkoholäther, Fettamime;

als organische Lösungsmittel, z. B. Alkohole, wie Äthanol, Butanole, Dimethylformamid, Dimethylsulfoxid, N-Methylpyrolidon, Aromaten, wie Toluol und Xylole;

als inerte Bestandteile, z. B. Kaolin, China-Clay, Talkum, Calciumcarbonat, hochdisperse Kieselsäure, Kieselgele, Kieselgur, Diatomenerde, Bims, Maisschrot, Verdickungsmittel, wie Stärke und Carboxymethylcellulose;

als Bindemittel, z. B. Magnesiumsulfat, Gips, Gummi-arabicum.

Beispielsweise werden die erfindungsgemäßen Wirkstoffe wie folgt formuliert:

1. Emulsionskonzentrat:

| | |
|---|---|
| 20 Gew.-% | Wirkstoff |
| 10 Gew.-% | handelsübliches epoxyliertes Anhydrosorbitmonolaurat (Handelsname "Tween Twenty") |
| 70 Gew.-% | Dimethylformamid |

2. Spritzpulver:

| | |
|---|---|
| 20 Gew.-% | Wirkstoff |
| 5 Gew.-% | Ammoniumligninsulfonat (Handelsname "Totanin") |
| 10 Gew.-% | Natriumoleylmethyltaurid (Handelsname "Arcopon T KONZ") |
| 65 Gew.-% | Kaolin. |

Die Aufwandmengen an Wirkstoffen können in großen Bereichen variieren. Bei der Saatgutbehandlung werden im allgemeinen Wirkstoffmengen von 0,05 bis 25 g/kg Saatgut benötigt.

Die Ausbringung der erfindungsgemäßen Wirkstoffe kann in jeder geeigneten Form erfolgen. Beispielhaft genannt seien Gießen, Verspritzen, Versprühen, Verstäuben, Bestreichen, Behandeln des Saatgutes (Beizen).

Die Erfindung wird nun anhand von Beispielen näher erläutert:

**Beispiel 1**

Herstellung vom 4-Chlorphenyl-alpha-bromessigsäure-3-jodpropargylester

a) Zu 18,9 g (0.1 Mol) 4-Chlorphenylessigsäurechlorid wurden bei 95°C 16 g (0.1 Mol) elementarem Broms unter Rühren getropft. Das Reaktionsgemisch wurde noch 2,5 h auf einer Temperatur von 90°C gehalten und anschließend überschüssiges Brom abdestilliert. Das Rohprodukt wurde schließlich unter 16 mb im Temperaturbereich von 90°C bis 96°C destilliert. Es wurden 22,9 g (0.085 Mol) 4-Chlorphenyl-alpha-bromessigsäurechlorid, entsprechend 85 % der Theorie, erhalten.

b) Zu einer Lösung von 5,46 g (0,03 Mol) 3-Jodpropinol und 5 g Pyridin in 20 ml absolutem Toluol wurde eine Lösung von 8,04 g (0.03 Mol) Chlorphenyl-alpha-bromessigsäurechlorid (gemäß a) in 20 ml absolutem Toluol unter Rühren getropft. Danach wurde das Reaktionsgemisch in Diäthyläther/Wasser aufgenommen und schließlich die organische Phase abgetrennt. Die organische Phase wurde noch mit 1 n Salzsäure und anschließend mit gesättigter Natriumhydrogencarbonat-Lösung extrahiert. Nach Trocknung mit Natriumsulfat und Abdestillieren des Lösungsmittels verblieben 9,5 g (0.023 Mol) an 4-Chlorphenyl-alpha-bromessigsäure-3-jodpropargylester, entsprechend 77 % der Theorie. Das Öl wird durch die folgenden NMR-Daten charakterisiert:

δ (80 MHz, $CDCl_3$ = 4,31 (s, 2H); 5.26 (s,H)
7.21 (AB, $J_{AB}$ = 10 Hz, 2H)
7.34 (AB, $J_{AB}$ = 10 Hz, 2H [ppm]

**Beispiel 2:**

Herstellung von 2.4-Dimethylphenylessigsäurejodpropargylester

a) In ein Gemisch aus 106 g m-Xylol, 531 g 16 n Salzsäure und 39 g Paraformaldehyd wurde bei 70°C 5 h lang Chlorwasserstoff eingeleitet. Danach wurde die organische Phase abgetrennt und destilliert. Es wurden im Siedebereich von 96°C - 100°C bei 16 mb Druck 105 g (0.68 Mol) 2.4-Dimethylbenzylchlorid, entsprechend 65 % der Theorie, erhalten.

b) In ein Gemisch aus 103 g (0.67 Mol) 2.-Dimethylbenzylchlorid (gemäß a) und 86 g (1.32 Mol) Kaliumcyanid in 50 ml Wasser wurden unter Rühren 10 g Tetrabutylammoniumbromid eingetragen. Bei der exothermen Reaktion erwärmte sich das Reaktionsgemisch auf 60°C. Nach Abklingen der Reaktion wurde die organische Phase abgetrennt und destilliert. Es wurden im Siedebereich von 86°C bis 91°C bei 0.07 mb Druck 89 g (0.61 Mol) an 2.4-Dimethylbenzylcyanid, entsprechend 94 % der Theorie, erhalten.

c) 85 g (0.59 Mol) 2.4-Dimethylbenzylcyanid (gemäß b) wurden bei 150°C 5 h lang in 50 Gew.%-iger Schwefelsäure gerührt. Danach wurde das Reaktionsgemisch auf Eis gegossen. Anschließend wurde mit 2 n Natronlauge alkalisch gestellt und schließlich die wäßrige Phase mit Äther extrahiert. Nach Abtrennen der ätherischen Phase wurde die wäßrige Phase mit 2 n Salzsäure angesäuert und die als Niederschlag anfallende Carbonsäure im Äther aufgenommen. Nach Abziehen des Äthers wurden 69 g (0.42 Mol) 2.4-Dimethylphenylessigsäure, entsprechend 71 % der Theorie, erhalten.

d) Eine Lösung von 16,4 g (0.10 Mol) 2.4-Dimethylphenylessigsäure (gemäß c) und 11 ml Thionylchlorid in 50 ml Cyclohexan wurde bis zur Beendigung der Gasentwicklung auf einer Temperatur von 80°C gehalten. Danach wurde überschüssiges Thionylchlorid zusammen mit Cyclohexan abdestilliert. Das verbliebene Säurechlorid wurde in 20 ml Toluol gelöst und in ein Gemisch aus 20,2 g (0.11 Mol) 3-Jodpropinol und 15 g Triäthylamin in 100 ml Toluol getropft. Die Reaktionstemperatur betrug 50°C. Das Reaktionsgemisch wurde schließlich in 100 ml Diäthyläther aufgenommen, danach mit 1 n Salzsäure und anschließend mit gesättigter Natriumhydrogencarbonatlösung extrahiert. Schließlich wurden die Phasen getrennt und die organische Phase mit Natriumsulfat getrocknet. Nach Abdestillieren des Äthers wurden 29,5 g (0.09 Mol) 2.4-Dimethylphenylessigsäurejodpropargylester, entsprechend 90 % der Theorie, erhalten.
Schmelzpunkt 39 - 40°C.

**Beispiel 3:**

Herstellung von 2.6-Dichlor-3-nitrophenylessigsäure-3-jodpropargylester

a) 29 g (0.14 Mol) 2.6-Dichlorphenylessigsäure wurden unter Rühren in ein Gemisch aus 40 ml 80 Gew.%-iger Salpetersäure und 20 ml 96 Gew.%-iger Schwefelsäure eingetragen. Die Reaktionstemperatur wurde im Bereich von 0 bis 5°C gehalten. Nach 1 h wurde die Reaktionsmischung auf 400 g Eis gegossen. Das Zielprodukt fiel als Niederschlag an, das abfiltriert und mit Wasser mineralsäurefrei gewaschen wurde. Es

wurden 32,3 g (0.13 Mol) an 2.6-Dichlor-3-nitrophenylessigsäure, entsprechend 92 % der Theorie, erhalten.

b) 15 g (0.06 Mol) 2.6-Dichlor-3-nitrophenylessigsäure wurden in 30 ml Cyclohexan und 20 ml Thionylchlorid suspendiert. Es wurde bis zur Beendigung der Gasentwicklung am Rückfluß gekocht. Danach wurde überschüssiges Thionylchlorid und Cyclohexan abdestilliert. Das erhaltene Säurechlorid wurde in 50 ml absolutem Toluol gelöst und unter Rühren bei einer Temperatur von 30° C zu einem Gemisch aus 11 g (0.06 Mol) 3-Jodpropinol, 4,75 g Pyridin und 50 g Toluol getropft. Nach 1 h wurde das Reaktionsgemisch in Diäthyläther aufgenommen und die ätherische Lösung mit 1 n Salzsäure und anschließend mit gesättigter Natriumhydrogencarbonatlösung extrahiert. Schließlich wurde die organische Phase mit Wasser gewaschen, mit Natriumsulfat getrocknet und eingedampft. Das Rohprodukt (22,7 g) wurde noch chromatographisch an Kieselgel gereinigt. Es wurden 15,5 g (0.033 Mol) an 2.6-Dichlor-3-nitrophenylessigsäurejodpropargylester, entsprechend 63 % der Theorie, erhalten.
Schmelzpunkt 97 - 99° C.

**Beispiel 4:**

Herstellung von alpha-Chlorphenylessigsäurejodpropargylester

a) 100 g (0,65 Mol) Mandelsäure, 234 g (1,95 Mol) Thionylchlorid wurden in 170 ml Cyclohexan gelöst und bis zur Beendigung der Gasentwicklung unter Rückfluß gekocht. Danach wurden überschüssiges Thionylchlorid und Cyclohexan abdestilliert. Das verbliebene Rohprodukt wurde fraktioniert destilliert. Es wurden im Siedeintervall von 103 bis 109° C bei 16 mb Druck alpha-Chlor-phenylessigsäurechlorid in einer Ausbeute von 75,7 g (0.40 Mol), entsprechend 61 % der Theorie, erhalten.

b) 24,6 g alpha-Chlor-phenylessigsäurechlorid, gelöst in 20 ml Toluol, wurden in ein Gemisch aus 23,7 g 3-Jodpropinol, 15 g Triäthylamin und 100 ml Toluol getropft. Die Reaktionstemperatur betrug 60° C. Das Reaktionsgemisch wurde schließlich in 100 ml Äther aufgenommen und die ätherische Lösung anschließend zunächst mit 1 n Salzsäure und anschließend mit gesättigter Natriumhydrogencarbonatlösung extrahiert. Zum Schluß wurde die organische Phase mit Natriumsulfat getrocknet. Nach Abdestillieren des Äthers wurden 36,5 g, entsprechend 84 % der Theorie, an alpha-Chlorphenylessigsäurejodpropargylester, erhalten.

Das Produkt wird durch die folgenden NMR-Daten charakterisiert:

α-Chlorphenylessigsäurejodpropargylester
δ (80 MHz, $CDCl_3$) = 4.81 (s, 2H); 5.31 (s, 1H)
7.1 - 7.5 (m, 5H).

**Beispiel 5:**

Sporenkeimtest

50 µl einer Lösung oder Suspension eines erfindungsgemäßen Wirkstoffs wurden zusammen mit 50 µl einer Sporensuspension, hergestellt durch Abschwemmen der Sporen von einer Agarkultur mit einer Nährlösung, die pro l 10 g Zucker, 1 g Glykol, 1 g $KH_2PO_4$ und 0,5 g $MgSO_4$ enthielt, in den Hohlschliff von Hohlschliffobjektträgern eingebracht.

Die Objektträger wurden bei 20° C 48 h in einer Petrischale, denen Boden mit einem angefeuchten Filterpapier bedeckt war, aufbewart.

Danach wurde das Verhältnis der gekeimten und der nicht gekeimten Sporen gegen eine unbehandelte Kontrollprobe verglichen.

Der Wirkungsgrad wurde in % nach der folgenden Formel berechnet:

$$100 - \frac{\text{Anzahl der gekeimten Sporen, behandelt}}{\text{Anzahl der gekeimten Sporen, unbehandelt}} \times 100$$

Als Wirkstoffe wurden verwendet:

A 4-Chlorphenylessigsäurejodpropargylester
B α-Brom-(4-Chlorphenyl)-essigsäurejodpropargylester
C 2.6-Dichlor-phenylessigsäurejodpropargylester
D α-Chlor-phenylessigsäurejodpropargylester
E 2.4-Dichlor-5-Nitro-phenylessigsäurejodpropargylester
F 4-Nitrophenylessigsäurejodpropargylester
G 2.4-Dichlor-3.5-dinitrophenylessigsäurejodpropargylester
H 4-Chlor-3.5-dinitrophenylessigsäurejodpropargylester
V Phenylessigsäurejodpropargylester als Vergleichssubstanz gemäß Stand der Technik.

Die Erbebnisse sind in der folgenden Tabelle 1 dargestellt.

**Tabelle 1**

Fungitoxizität gegen Pilzsporen

| Wirkstoff | Wirkstoffkonzentration in [ppm] | Alternaria tenuis | Botrytis cinerea | Fusarium culmorum | Fusarium nivale | Colletotrichum coffeanum | Verticillium dahliae | Penicillium glaucum |
|---|---|---|---|---|---|---|---|---|
| A | 4 | 90 | 90 | 95 | 90 | 95 | 95 | 95 |
| | 2 | 80 | 80 | 95 | 80 | 90 | 80 | 95 |
| | 1 | 80 | 80 | 90 | 60 | 80 | 80 | 90 |
| B | 4 | 100 | 100 | 100 | 100 | 100 | 100 | 100 |
| | 2 | 100 | 100 | 100 | 100 | 100 | 100 | 100 |
| | 1 | 100 | 100 | 100 | 100 | 100 | 100 | 100 |
| C | 4 | 95 | 100 | 95 | 100 | 95 | 100 | 90 |
| | 2 | 95 | 95 | 95 | 100 | 95 | 100 | 90 |
| | 1 | 95 | 95 | 95 | 100 | 95 | 100 | 60 |
| D | 4 | 90 | 90 | 95 | 90 | 95 | 95 | 95 |
| | 2 | 90 | 80 | 95 | 80 | 90 | 80 | 95 |
| | 1 | 80 | 80 | 90 | 60 | 80 | 80 | 90 |
| E | 4 | 100 | 40 | 100 | 100 | 100 | 40 | 100 |
| | 2 | 100 | 30 | 100 | 100 | 100 | 30 | 100 |
| | 1 | 100 | 30 | 100 | 100 | 100 | 30 | 100 |
| F | 2 | 100 | 40 | 40 | 80 | 90 | 90 | 80 |
| G | 2 | 70 | 100 | 70 | 80 | 60 | 70 | 70 |
| H | 2 | 100 | 70 | 90 | 90 | 100 | 40 | 100 |
| V | 4 | 100 | 50 | 40 | 90 | 80 | 30 | 40 |
| | 2 | 90 | 0 | 0 | 70 | 30 | 0 | 0 |
| | 1 | 80 | 0 | 0 | 60 | 20 | 0 | 0 |

Bei einem Vergleich der Ergebnisse, die mit erfindungsgemäßen Wirkstoffen erzielt wurden, mit denen der Vergleichssubstanz (V) zeigt sich die erhöhte fungizide Wirksamkeit (z. B. gegen Botrytis cinerea) und insbesondere ein deutlich verbreitetes Wirkungsspektrum.

**Beispiel 6:**

Fungizide Wirksamkeit gegen Botrytis cinerea an Puffbohnen

Es wurden Puffbohnen der Sorte "Midget" bis zu einer Wuchshöhe von ca. 10 cm herangezogen. Die Pflanzen wurden bis zur Tropfnässe mit Spritzbrühen besprüht, die einen Gehalt an 300 ppm Wirksubstanz aufweisen.

Nach Antrocknen des Spritzbelages wurden die Pflanzen mit einer Sporensuspension von Botrytis cinerea inokuliert. Die Aufstellung der Pflanzen erfolgte im Klimaschrank bei konstant 20°C und einer Luftfeuchte von 95 %.

Nach 6 Tagen wurde auf Befall bonitiert. Die Wirksamkeit wurde im Vergleich zu unbehandelten Proben ermittelt.

Der Wirkungsgrad in % wurde nach der folgenden Formel ermittelt:

$$\% = (1 - \frac{\text{befallene Fläche, behandelt}}{\text{befallene Fläche, unbehandelt}} = x\ 100$$

Als Wirkstoffe wurden eingesetzt:

I 2-Chlor-3.5-dinitrophenylessigsäurejodpropargylester
K 2.6-Dichlor-3.5-dinitrophenylessigsäurejodpropargylester

L 2.4.6-Trimethylphenylessigsäurejodpropargylester
H 4-Chlor-3.5-dinitrophenylessigsäurejodpropargylester
V Phenylessigsäurejodpropargylester als Vergleichssubstanz gemäß Stand der Technik.

Die Ergebnisse sind in der folgenden Tabelle 2 zusammengestellt.

**Tabelle 2**

Wirksamkeit gegen Botrytis cinerea an Puffbohnen bei 500 ppm Wirkstoffkonzentration

| Wirkstoff | Wirksamkeit in % |
|---|---|
| I | 80 |
| K | 30 |
| L | 20 |
| H | 30 |
| V | 0 |

**Patentansprüche** für die Vertragsstaaten: BE, CH, DE, FR, GB, IT, LI, NL, SE

1. Verbindungen der allgemeinen Formel

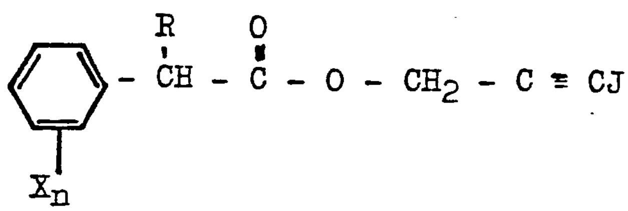

wobei

R Wasserstoff, Fluor, Chlor, Brom, Jod, Methoxy, 1-Imidazolyl und 1.2.4-Triazolyl bedeutet,
X für einen oder mehrere gleiche oder verschiedene Substituenten steht mit der Bedeutung Fluor, Chlor, Brom, Jod, Cyano, Nitro, Carboxy, Alkyl mit 1 bis 12 Kohlenstoffatomen, Alkoxy mit 1 bis 12 Kohlenstoffatomen, Cycloalkyl mit 3 bis 6 Kohlenstoffatomen, Formyl, Acetyl, Propionyl, Benzoyl, Phenylsulfonyl, Phenyl, Phenoxy und substituiertes Phenyl und Phenoxy mit jeweils 1 bis 3 Substituenten aus der Gruppe Fluor, Chlor, Brom, Nitro, Methyl und Methoxy,
n eine ganze Zahl von 1 bis 5 ist und mit der Maßgabe, daß R nicht Wasserstoff bedeutet, 0 sein kann.

2. Fungizid wirksame Zusammensetzungen, die mindestens 1 Verbindung nach Anspruch 1 enthalten.
3. Verfahren zur Herstellung von Verbindungen nach Anspruch 1, dadurch gekennzeichnet, daß Verbindungen der Formel

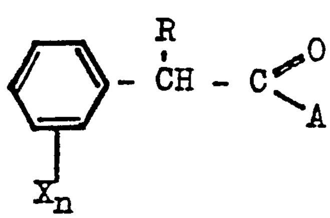

wobei
R, X und n die gemäß Anspruch 1 gegebene Bedeutung besitzen und
A für Chlor, Brom, Jod, Acyloxy und Alkoxycarbonyloxy steht,
mit 3-Jod-propinol in Gegenwart eines Säurebindemittels umgesetzt werden.
4. Verwendung von Verbindungen nach Anspruch 1 als fungizide Mittel.

**Patentansprüche** für den Vertragsstaat: AT

1. Verfahren zur Herstellung von Verbindungen der allgemeinen Formel

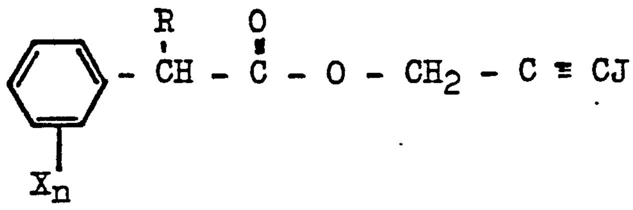

wobei

R Wasserstoff, Fluor, Chlor, Brom, Jod, Methoxy, 1-Imidazolyl und 1.2.4-Triazolyl bedeutet,
X für einen oder mehrere gleiche oder verschiedene Substituenten steht mit der Bedeutung Fluor, Chlor, Brom, Jod, Cyano, Nitro, Carboxy, Alkyl mit 1 bis 12 Kohlenstoffatomen, Alkoxy mit 1 bis 12 Kohlenstoffatomen, Cycloalkyl mit 3 bis 6 Kohlenstoffatomen, Formyl, Acetyl, Propionyl, Benzoyl, Phenylsulfonyl, Phenyl, Phenoxy und substituiertes Phenyl und Phenoxy mit jeweils 1 bis 3 Substituenten aus der Gruppe Fluor, Chlor, Brom, Nitro, Methyl und Methoxy,
n eine ganze Zahl von 1 bis 5 ist und mit der Maßgabe, daß R nicht Wasserstoff bedeutet, 0 sein kann,

dadurch gekennzeichnet, daß Verbindungen der Formel

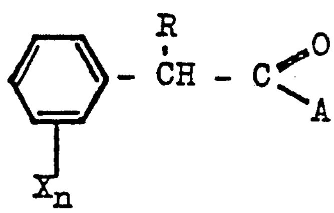

wobei
R, X und n die obige Bedeutung besitzen und
A für Chlor, Brom, Jod, Acyloxy und Alkoxycarbonyloxy steht,
mit 3-Jod-propinol in Gegenwart eines Säurebindemittels umgesetzt werden.

2. Verwendung einer nach Anspruch 1 erhaltenen Verbindung als Wirkstoff in einem fungiziden Mittel.

**Claims** for the Contracting States: BE, CH, DE, FR, GB, IT, LI, NL, SE

1. Compounds of the general formula

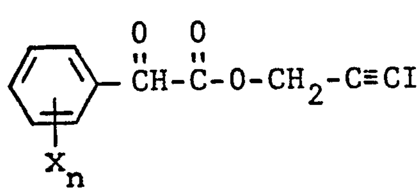

whereby R signifies hydrogen, fluorine, chlorine, bromine, iodine, methoxy, 1-imidazolyl and 1,2,4-triazolyl, X stands for one or more identical or different substituents with the meaning fluorine, chlorine, bromine, iodine, cyano, nitro, carboxyl, alkyl with 1 to 12 carbon atoms, alkoxy with 1 to 12 carbon atoms, cycloalkyl with 3 to 6 carbon atoms, formyl, acetyl, propionyl, benzoyl, phenylsulphonyl, phenyl, phenoxy and substituted phenyl and phenoxy with, in each case, 1 to 3 substituents from the group fluorine, chlorine, bromine, nitro, methyl and methoxy, n is a whole number from 1 to 5 and, with the proviso that R does not signify hydrogen, can be 0.

2. Fungicidally-effective compositions which contain at least 1 compound according to claim 1.

3. Process for the preparation of compounds according to claim 1, characterised in that compounds of the formula

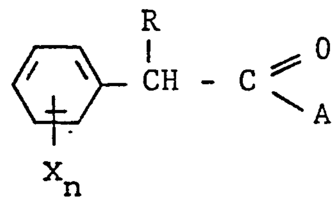

whereby R, X and n possess the meaning according to claim 1 and A stands for chlorine, bromine, iodine, acyloxy and alkoxycarbonyloxy, are reacted with 3-iodopropargyl in the presence of an acid-binding agent.

4. Use of compounds according to claim 1 as fungicidal agents.

**Claims** for the Contracting State: AT

1. Process for the preparation of compounds of the general formula

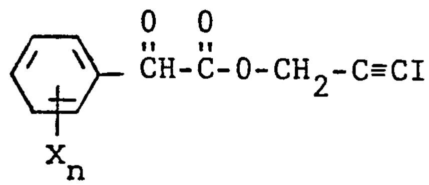

whereby R signifies hydrogen, fluorine, chlorine, bromine, iodine, methoxy, 1-imidazolyl and 1,2,4-triazolyl, X stands for one or more identical or different substituents with the meaning fluorine, chlorine, bromine, iodine, cyano, nitro, carboxyl, alkyl with 1 to 12 carbon atoms, alkoxy with 1 to 12 carbon atoms, cycloalkyl with 3 to 6 carbon atoms, formyl, acetyl, propionyl, benzoyl, phenylsulphonyl, phenyl, phenoxy and substituted phenyl and phenoxy with, in each case, 1 to 3 substituents from the group fluorine, chlorine, bromine, nitro, methyl and methoxy, n a whole number of 1 to 5 and, with the proviso that R does not signify hydrogen, can be 0, characterised in that compounds of the formula

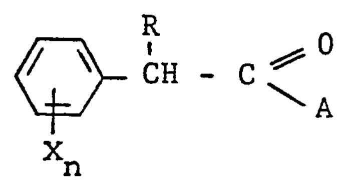

whereby R, X and n possess the above meaning and A stands for chlorine, bromine, iodine, acyloxy and alkoxycarbonyloxy, are reacted with 3-iodopropynol in the presence of an acid-binding agent.

2. Use of a compound obtained according to claim 1 as active material in a fungicidal agent.

**Revendications** pour les Etats contractants: BE, CH, DE, FR, GB, IT, LI, NL, SE

1. Composes de formule générale

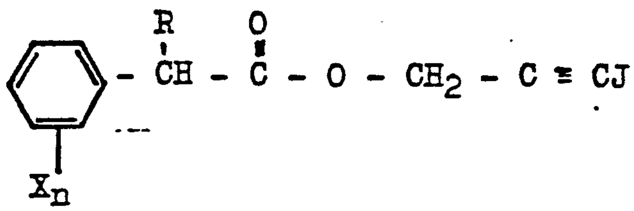

dans laquelle

R représente un atome d'hydrogène, un atome de fluor, un atome de chlore, un atome de brome, un atome d'iode, un groupe méthoxy, un groupe 1-imidazolyle et un groupe 1,2,4-triazolyle,

X représente un ou plusieurs substituants identiques ou différents, signifiant un atome de fluor, un atome de chlore, un atome de brome, un atome d'iode, un groupe cyano, un groupe nitro, un groupe carboxyle, un groupe alkyle comportant de 1 à 12 atomes de carbone, un groupe alcoxy comportant de 1 à 12 atomes de carbone, un groupe cycloalkyle comportant de 3 à 6 atomes de carbone, un groupe formyle, un groupe acétyle, un groupe propionyle, un groupe benzoyle, un groupe phénylsulfonyle, un groupe phényle, un groupe phénoxy et un groupe phényle ou phénoxy substitué, avec chaque fois 1 à 3 substituants choisis dans le groupe constitué par les atomes de fluor, chlore, brome, et les groupes nitro, méthyle et méthoxy,

n représente un nombre entier de 1 à 5, et, à condition que R ne représente pas un atome d'hydrogène, peut être 0.

2. Compositions à activité fongicide, comprenant au moins un composé selon la revendication 1.

3. Procédé pour la préparation des composés selon la revendication 1, caractérisé en ce qu'on fait réagir des composés de formule

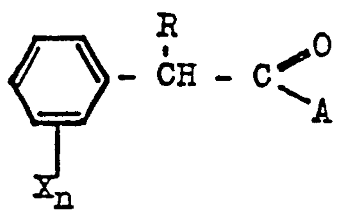

dans laquelle

R, X et n ont la signification mentionnée dans la revendication 1, et

A représente un atome de chlore, un atome de brome, un atome d'iode, un groupe acyloxy et un groupe alcoxycarbonyloxy, avec le 3-iodo-propynol, en présence d'un agent fixant les acides.

4. Utilisation des composés selon la revendication 1, comme agent fongicide.

**Revendications** pour l'Etat contractant: AT

1. Procédé pour la préparation des composés de formule générale

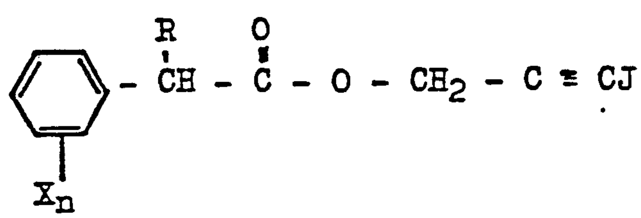

dans laquelle

R représente un atome d'hydrogène, un atome de fluor, un atome de chlore, un atome de brome, un atome d'iode, un groupe méthoxy, un groupe 1-imidazolyle et un groupe 1,2,4-triazolyle,

X représente un ou plusieurs substituants identiques ou différents, signifiant un atome de fluor, un atome de chlore, un atome de brome, un atome d'iode, un groupe cyano, un groupe nitro, un groupe carboxyle, un groupe alkyle comportant de 1 à 12 atomes de carbone, un groupe alcoxy comportant de 1 à 12 atomes de

carbone, un groupe cycloalkyle comportant de 3 à 6 atomes de carbone, un groupe formyle, un groupe acétyle, un groupe propionyle, un groupe benzoyle, un groupe phénylsulfonyle, un groupe phényle, un groupe phénoxy et un groupe phényle ou phénoxy substitué, avec chaque fois 1 à 3 substituants choisis dans le groupe constitué par les atomes de fluor, chlore, brome, et les groupes nitro, méthyle et méthoxy,

n représente un nombre entier de 1 à 5, et, à condition que R ne représente pas un atome d'hydrogène, peut être 0,

caractérisé en ce qu'on fait réagir des composés de formule

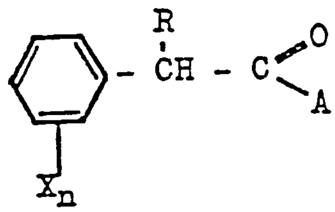

dans laquelle

R, X et n ont la signification mentionnée précédemment, et

A représente un atome de chlore, un atome de brome, un atome d'iode, un groupe acyloxy et un groupe alcoxycarbonyloxy,

avec le 3-iodo-propynol, en présence d'un agent fixant les acides.

2. Utilisation d'un des composés obtenus selon la revendication 1, comme agent actif dans un produit fongicide.